# FASCICULE DE BREVET EUROPEEN

(11) **EP 0 586 286 B1**
(45) Date de publication et mention de la délivrance du brevet: **02.07.1997**
(21) Numéro de dépôt: 93402090.0
(22) Date de dépôt: 24.08.1993
(51) Int. Cl.: A61B 5/00

(54) **Système de transmission de données médicales, sans fil**
Drahtloses Übertragungssystem für medizinische Daten
Wireless transmission system for medical data

(30) Priorité: 04.09.1992 FR 9210568
(43) Date de publication de la demande: 09.03.1994
(73) Titulaire: THOMSON-CSF, 75008 Paris (FR)
(72) Inventeur: Hethuin, Serge, F-92402 Courbevoie Cedex (FR)
(74) Mandataire: Lincot, Georges

(56) Documents cités:
- EP-A- 0 347 515
- WO-A-90/09143
- WO-A-91/16851
- CH-A- 675 675
- US-A- 3 902 478
- US-A- 4 958 645

## Description

L'invention se rapporte au domaine de l'électronique médicale et a plus particulièrement pour objet un système de transmission de données médicales sans fil.

Certains examens médicaux utilisent la détection de champs électriques. C'est notamment le cas en électrocardiographie.

Classiquement pour effectuer un électrocardiogramme, des électrodes sont disposées en des points précis du corps et les tensions détectées entre ces points sont amplifiées dans un amplificateur différentiel de caractéristiques adaptées, suivi d'un enregistreur graphique. Dans les ensembles utilisés pour la surveillance, l'enregistreur graphique est remplacé ou complété par un oscilloscope. La valeur diagnostique de l'électrocardiogramme ou "E.C.G". est considérable : les altérations des formes d'ondes enregistrées sont très significatives.

Classiquement, pour des examens complets destinés à caractériser complètement l'activité électrique du coeur en 3 dimensions, 3 signaux de caractérisation sont formés à partir de 10 électrodes : 3 électrodes dites "principales", D, G, P, 1 électrode de référence, et 6 électrodes dites "précordiales". Les patients sont allongés et des fils relient les électrodes disposées sur le corps aux circuits électroniques de l'électrocardiographe. Cette liaison est par exemple effectuée de la manière suivante : 10 câbles élémentaires sont reliés aux 10 électrodes disposées aux points requis pour l'examen. Ces 10 câbles sont reliés à un concentrateur par des fiches types "banane" et un cordon blindé multiconducteurs relie le concentrateur a l'enregistreur via une fiche DIN. Cette structure est lourde, chère, contraignante pour le patient et sa sécurité de fonctionnement n'est même pas complètement assurée puisque des ruptures ou des anomalies dans les liaisons filaires, notamment lorsque l'âme centrale d'un câble est endommagée, produisent du bruit et des artefacts, voire une disparition du signal correspondant.

De plus, si la position allongée est convenable pour certaines mesures elle ne l'est plus pour des mesures pendant des épreuves d'effort. Or ces examens ont un grand intérêt pour suivre les variations des signaux E.C.G. en fonction de l'effort. Dans les systèmes classiques, le patient est sur un "home trainer" et les problèmes de vibration, de sudation accentuent les déficiences des câbles. De plus l'équipement, gênant, porté par le patient le pénalise lors de son effort.

D'autres équipements, de type portable, sont prévus pour enregistrer les signaux E.C.G. sur un magnétophone porté à la ceinture et relié par câbles aux électrodes. De tels équipements utilisables en extérieur pour la surveillance de patients tout au long de leur activité peuvent permettre d'enregistrer tous les signaux pendant 12H ou 24H pour relever des anomalies, liées à l'activité, indécelables lors d'examens en cabinet. Un tel équipement ne permet pas une surveillance du patient en temps réel mais seulement en temps différé après lecture, éventuellement accélérée, de la bande.

On connaît également un équipement de test particulièrement destiné aux sportifs notamment pour améliorer leur entraînement. Cet équipement comporte un émetteur placé sur la poitrine du sportif qui détecte les dépassements par rapport à un seuil (points "R" du signal complexe E.C.G. PQRST), et transmet sans fil, des impulsions correspondant à ces points, à un micro-ordinateur récepteur porté au poignet. Cette transmission sans fil s'effectue selon le principe du champ magnétique ; l'eau, les vêtements ou le corps mouillés ne perturbent pas la transmission. L'équipement permet d'enregistrer la fréquence cardiaque selon un prélèvement au rythme de 5, 15 ou 60s par exemple pendant une durée totale fonction des capacités de mémorisation.

Comme le montre la description succincte donnée ci-dessus, cet équipement ne permet de suivre que la fréquence cardiaque, et l'utilisation des résultats ne peut être faite en temps réel que par le sportif et seulement dans la mesure où un afficheur peut être prévu sur le récepteur. Comme dans l'équipement décrit avant, un tel équipement ne permet pas un suivi en temps réel par une équipe médicale.

Un autre système de transmission de signaux ECG par voie radio est également connu de la demande de brevet W090/09143. Ce système permet de différencier les différents signaux transmis par un codage numérique qui ne permet cependant pas d'éliminer des interférences qui peuvent se produire entre plusieurs systèmes du même type.

Il est également connu du brevet US 4 958 645 une chaîne de télémétrie permettant la transmission de données médicales mesurées sur le corps de patient, où chaque donnée est associée à un code d'identification de chaque patient. Cette chaîne de transmission qui est entièrement numérique est onéreuse à réaliser et n'offre pas toute les sécurités nécessaires à une bonne transmission.

L'invention a pour objet un système de transmission de données médicales qui ne présente pas les inconvénients des systèmes utilisant des liaisons par fils et qui permette de faire suivre en temps réel et éventuellement à distance les signaux E.C.G d'au moins un patient sous surveillance, par une équipe médicale, sans limitation des paramètres observés. Dans les modes de réalisation les plus perfectionnés il est possible de suivre à distance une cinquantaine de patients.

A cet effet, l'invention a pour objet un système de transmission de données médicales, sans fil pour la transmission entre émetteur/récepteur de signaux électriques caractéristiques résultant d'un champ électrique entre électrodes disposées sur le corps du patient, comprenant un équipement d'émission à maintenir sur le corps du patient par des moyens amovibles, relié à deux électrodes et comportant un circuit d'amplification des signaux électriques coupé à un modulateur d'une porteuse à la fréquence d'émission, caractérisé en ce que, pour sécuriser le canal de transmission entre émetteur/récepteur et autoriser un fonctionnement multicanaux, l'équipement d'émission comporte un circuit codeur de canal couplé d'une part à la sortie du circuit d'amplification et d'autre part à un générateur de code qui génère un code pseudo-aléatoire associé au canal de liaison affecté à l'équipement d'émission, pour coder le signal analogique utile avant transmission au modulateur.
- La figure 1, illustre un premier mode de réalisation de l'équipement d'émission selon l'invention.
- La figure 2, illustre un deuxième mode de réalisation de l'équipement d'émission, dans un mode dit "semi-numérique".
- La figure 3, représente une période du signal E.C.G.
- La figure 4 illustre le signal issu du codeur de canal dans le mode de réalisation semi-numérique de l'équipement d'émission.

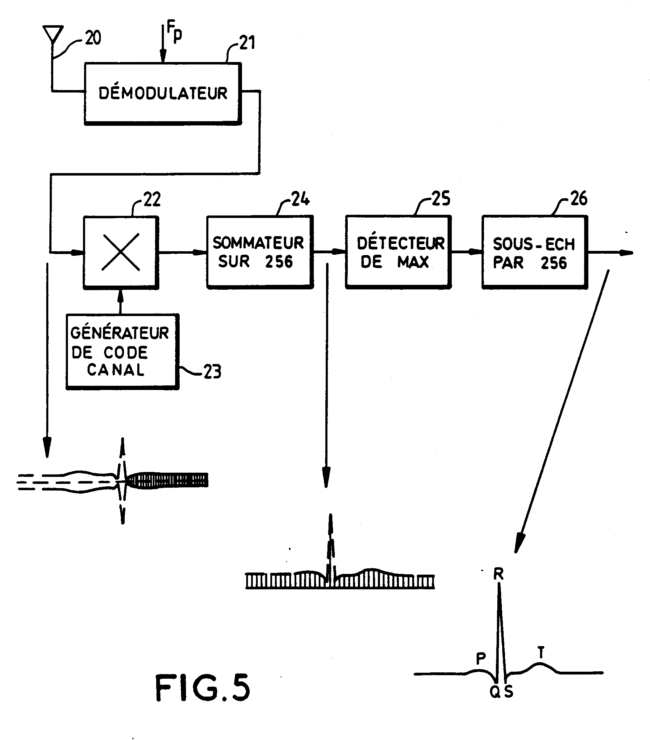

- La figure 5 illustre la partie non conventionnelle de l'équipement de réception associé au mode de réalisation semi-numérique de l'équipement d'émission.

Comme indiqué ci-dessus, selon l'invention, la transmission des données médicales, notamment les données de caractérisation de l'activité électrique du coeur, est effectuée non plus par fils mais par transmission des signaux électriques sur des ondes électromagnétiques porteuses pour éviter tous les problèmes liés à la connectique. Cependant il est essentiel pour la mise en oeuvre de telles transmissions, de tenir compte des impératifs de sécurité requis pour ce type d'équipements et des contraintes liées à l'environnement dans lequel ces systèmes doivent opérer. Les différentes gammes d'ondes, VLF, UHF, SHF, IR pourront être utilisées. Elles ont chacune des atouts mais aussi des contraintes d'utilisation. Les bandes à transmettre doivent être adaptées à l'onde porteuse associée. Enfin il est essentiel pour qu'une surveillance complète en temps réel, puisse être effectuée, que le signal complexe E.C.G. puisse être transmis dans sa totalité. Quelques rappels sur les caractéristiques de ce signal permettant de mieux comprendre le type de contraintes à considérer sont donnes ci-après : Les signaux électriques captés entre électrodes ont une amplitude de l'ordre du millivolt et une bande de l'ordre de 0,5 Hz à 500 Hz (fréquence du front de montée de l'impulsion "R"). Ces signaux utiles doivent être séparés des signaux à la fréquence secteur, (50 Hz en Europe), et de ses harmoniques, qui sont précisément dans la bande du signal utile, par filtrage mais sans que ce filtrage affecte les signaux utiles.

D'autre part, pour obtenir la sécurité nécessaire en cas de perturbation, les modes préférés de réalisation du système selon l'invention transmettent les données par modulation de fréquence ou de phase et dans certains cas permettent d'utiliser des codes correcteurs d'erreurs.

Enfin la transmission sans fil a pour avantage important qu'elle permet à une équipe médicale unique de surveiller plusieurs patients, chacun d'eux portant un émetteur qui transmet les signaux E.C.G. au centre de surveillance. Mais pour obtenir cette fonctlonnalité, il est indispensable que les perturbations entre équipements de même type soient minimisées, ce qui implique que les données soient aisément séparables, c'est-à-dire soient transmises sur des porteuses de fréquences différentes (sélection du canal de type FDMA) si l'on utilise une modulation analogique. Dans une modulation numérique on peut indistinctement utiliser une séparation par porteuses (type FDMA) ou une séparation par codes PN orthogonaux (type CDMA).

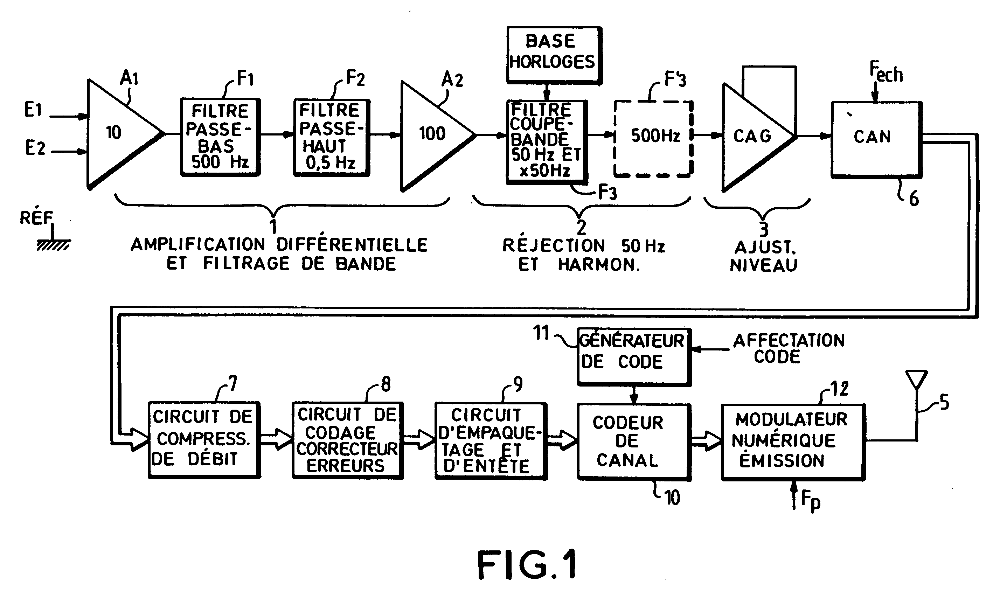

La figure 1 illustre l'équipement d'émission selon l'invention dans son mode de réalisation le plus simple :

Deux électrodes, soient E₁ et E₂ entre lesquelles on veut suivre la variation de tension électrique, sont reliées aux deux entrées d'un étage d'amplification différentielle A₁, l'électrode de référence Ref donnant le potentiel de masse de l'amplificateur. Le gain de cet étage est de l'ordre de 10. Un filtre passe-bas F₁ dont la fréquence de coupure est de l'ordre de 500 Hz est placé en sortie de cet étage d'amplification. Un filtre passe-haut F₂ dont la fréquence de coupure est de l'ordre de 0,5 Hz est relié à la sortie du filtre passe-bas et un amplificateur A₂ ayant un gain de l'ordre de 10 est place à sa sortie.

Ce premier ensemble, 1, d'amplification différentielle et filtrage de bande, permet de rejeter le mode commun, c'est-à-dire une grande partie des parasites captés simultanément par les deux électrodes utiles, et notamment du secteur à 50 Hz, et ne transmet que la bande du signal utile, entre 0,5 Hz et 500 Hz. Cependant il subsiste dans la bande des parasites à 50 Hz et aux harmoniques du 50 Hz. C'est pourquoi un deuxième ensemble de circuits 2, est prévu ensuite pour éliminer le plus complètement possible ces parasites et seulement ces parasites. Il comporte un filtre coupe bande F₃ présentant une atténuation très importante dans une bande étroite 0,4 Hz, autour de 50 Hz et des harmoniques. Pour obtenir ces caractéristiques, le mode préféré de réalisation de ce filtre F₃ est un filtre à aiguilles multi-harmoniques à n chemins, n étant choisi égal par exemple à 8. Dans ce cas un filtre passe bas F'₃ (illustré en pointillés) de fréquence de coupure 500 Hz est prévu pour couper les fréquences supérieures à 500 Hz générées par le filtre à aiguilles.

Le troisième ensemble, 3, est un circuit à amplificateur à commande automatique de gain pour que le signal appliqué aux circuits d'émission 4, qui suivent ait une dynamique convenable.

Cet équipement d'émission est porté par le patient et peut aisément être contenu dans un petit boîtier maintenu sur le thorax de la personne à surveiller par exemple au moyen d'une ceinture, les deux électrodes portées par la ceinture étant reliées à des bornes prévues sur le boîtier. Quand l'équipement est "analogique", ce qui a l'avantage de la simplicité et de la compacité, cela présente cependant l'inconvénient d'être sensible aux perturbations par des émissions extérieures et aux perturbations entre équipements de même type, qui devront émettre à des fréquences différentes.

Le signal E.C.G. à transmettre est numérisé au moyen d'un convertisseur analogique-numérique, 6, sur 12 bits par exemple, commandé à la fréquence d'échantillonnage F_{ech}, par exemple 1 KHz. Un circuit de compression de débit 7 relié à la sortie du convertisseur permet de baisser le débit de 12 kbits/s à 1 kbit/s.

L'équipement comporte ensuite, en série, un circuit de codage correcteur d'erreurs 8, pour correction à la réception des données reçues, par code type RS (Reed Solomon) ou BCH (Binary Coding Hamming), puis un circuit 9 de formation pour la mise en paquets des données avec création d'entête pour la synchronisation des messages, et enfin un codeur de canal 10 par un codage PN pseudo-aléatoire, de type "GOLD" ou "KASAMI". Le débit passe alors de 1 kbit/s à 256 kbit/s. Le code de type KASAMI permet de définir N = 16 canaux orthogonaux avec une longueur de code égale à 17x15 = 255 éléments. Pour cela un générateur de code 11 commande le circuit de codage canal 10. Le code propre à l'émetteur étant fixé par des clés permettant d'affecter à chaque émetteur un code particulier, de manière définitive ou reprogrammable suivant les applications.

Le signal numérique codé est alors appliqué à un modulateur numérique 12 qui reçoit par ailleurs la fréquence porteuse à moduler Fₚ, et transmet la porteuse modulée à l'antenne d'émission 5.

Le mode de réalisation précisément décrit ci-dessus, comporte un circuit de commande automatique de gain sur le signal analogique avant numérisation. Ce circuit n'est pas indispensable si la dynamique du convertisseur est suffisante. Dans ce cas la fonction C.A.G. peut être réalisée entièrement en numérique il n'y a plus alors d'amplification à commande automatique de gain, ou en numérique seulement pour l'élaboration de la commande, le signal de commande élaboré à partir du signal de sortie convertisseur servant alors à commander un amplificateur à gain variable situé avant le convertisseur.

De même le mode de réalisation décrit ci-dessus comporte un codeur de canal (10) et le générateur de code associé. Pour un système ne nécessitant qu'un canal liaison en simplex, un émetteur relié à un récepteur à courte distance, ces éléments peuvent être supprimés.

L'équipement, dans la version à modulation numérique décrite ci-dessus est très performant puisqu'il permet de mettre en oeuvre toutes les techniques numériques de correction d'erreurs et de séparation des canaux connues. Mais son niveau de sophistication peut être trop important notamment pour des applications où le moindre coût est un aspect essentiel.

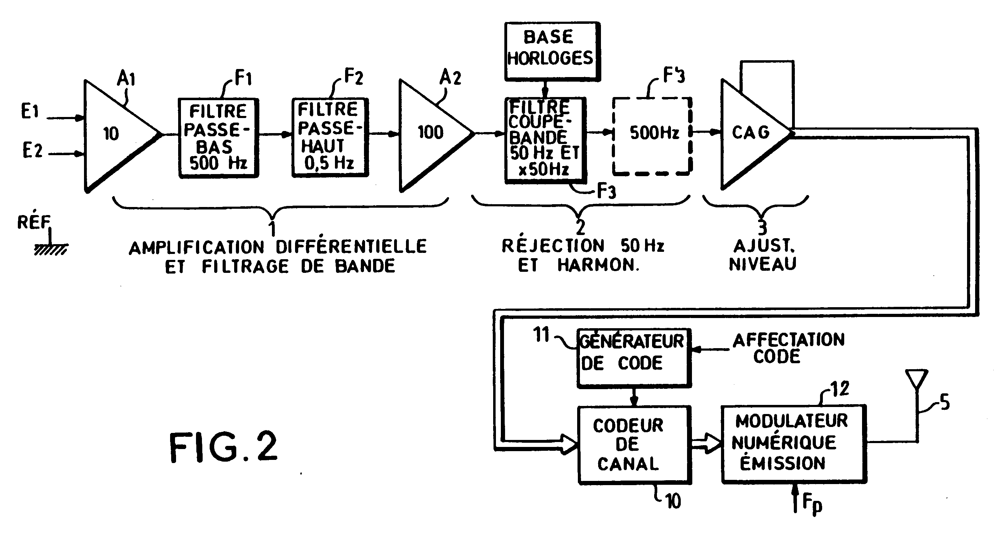

L'équipement représenté à la figure 2 comporte, comme précédemment, l'ensemble 1 d'amplification différentielle et filtrage de bande, l'ensemble 2 de réjection du 50 Hz et de ses harmoniques, l'ensemble 3 d'amplification avec commande automatique de gain, mais au lieu d'échantillonner le signal analogique pour obtenir un signal numérisé qui après traitement module une porteuse Fₚ, le signal analogique E.C.G. est directement modulé par un circuit 10 de codage canal par un code pseudo-aléatoire PN choisi entre plusieurs codes orthogonaux, pour permettre à la réception de distinguer le signal émis par cet émetteur d'un signal analogue émis par un émetteur voisin.

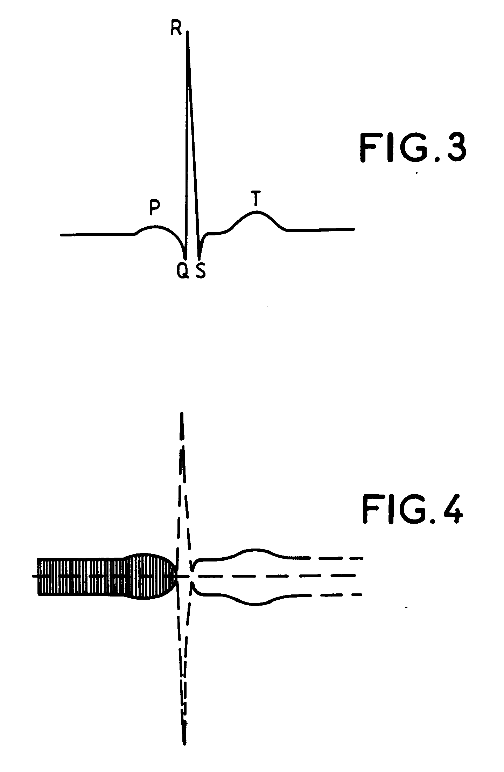

Les figures 3 et 4 permettent d'illustrer cette modulation.

La figure 3 illustre le signal E.C.G. avec ses points caractéristiques PQRST et la figure 4 illustre le signal codé après codage canal par code pseudo-aléatoire. Le code cyclique particulier au canal est répété chaque milliseconde, soit 1000 fois à peu prés sur la durée de l'ordre de 1s de l'onde E.C.G. Comme décrit ci-dessus pour une fréquence de changement d'état du code de 256 KHz, la longueur du code est L = 255 éléments obtenue avec un code sur 8 bits auxquels est ajoutée une période élémentaire pour la remise à zéro du générateur, ce qui permet de générer au plus 16 canaux orthogonaux par code de KASAMI.

Le signal ainsi codé est directement appliqué au modulateur 12 de la fréquence porteuse Fₚ. Ainsi comme précédemment des liaisons proches sont différenciées par les codes affectés à chacune d'elles même si leurs porteuses sont identiques (Technique dite "CDMA").

On obtient ainsi une version de l'équipement protégée de manière efficace et d'une complexité nettement moindre que celle de la version numérique décrite ci-dessus.

Bien sûr, cette version ne permet pas d'introduire un codage correcteur d'erreurs, mais permet cependant une pondération des résultats obtenus en fonction des maximums de corrélation.

Pour le premier mode de réalisation de l'équipement d'émission selon l'invention la station de réception correspondante comporte des moyens de réception de démodulation et, pour la version numérique de décodage et de restitution du signal analogique E.C.G. qui sont classiques. Pour la gestion de plusieurs émetteurs, elle comporte également les moyens de séparation des canaux nécessaires.

Pour le second mode de réalisation de l'équipement d'émission, semi-numérique, l'équipement de réception est adapté.

La restitution du signal à la réception est faite par synchronisation sur le code, c'est-à-dire à partir d'un "échantillon" de signal à la fréquence 1 KHz. En effet, il est nécessaire d'acquérir la synchronisation, c'est-à-dire de repérer les instants où le code attendu est présent, par corrélation avec la référence de code et de sous-échantillonner le signal ainsi construit en prélevant les échantillons successifs correspondant aux maximums successifs du signal de corrélation : le signal restitué est un signal à la cadence de 1 KHz.

Le schéma synoptique du récepteur associé à l'émetteur pour former le système selon l'invention est illustré sur la figure 6 où ont également été illustrés les signaux en trois points du récepteur : le signal reçu sur l'antenne de réception 20 est la porteuse modulée par le signal E.C.G. codé. Après démodulation et numérisation des voies en quadrature I et Q dans un démodulateur 21 recevant la fréquence porteuse Fₚ et correspondant au modulateur utilisé dans l'émetteur, ce signal est appliqué à un opérateur 22 qui reçoit par ailleurs le code affecté au canal correspondant à l'émetteur à surveiller d'un générateur de code 23. Un sommateur 24 sur 256 éléments, c'est-à-dire la longueur de code, permet ensuite, dans un détecteur de maximums 25 de détecter des maximums qui correspondent à une synchronisation entre le code de canal et les échantillons de signal successifs à l'entrée de l'opérateur. L'enveloppe de ces maximums obtenue après sous-échantillonnage par 256 dans un circuit 26 est le signal E.C.G., PQRST restitué.

Dans les modes de réalisation de l'émetteur décrits ci-dessus, il n'a pas été précisé comment la fréquence porteuse était générée. Elle peut être, de manière classique issue d'un générateur de porteuse 15 situé dans l'émetteur, comme illustré en pointillés sur la figure 1. Mais, dans un autre mode de réalisation, l'invention pourrait utiliser après élaboration du signal de modulation un système de transmission hyperfréquence dans lequel la station de surveillance S, à qui sont destinées les données élaborées dans l'équipement porté par le patient, émet à destination de cet équipement une onde porteuse hyperfréquence d'interrogation Fₚ, à 2,45 GHz par exemple.

L'équipement porté par le patient P₁, P₂ ... P₄ comporte une carte de modulation de l'onde reçue par les données à émettre avant renvoi vers la station.

L'invention, d'un domaine d'application très large, est adaptable aux différentes applications visées. En effet pour un équipement destiné à un cabinet cardiologique, il suffit d'une liaison unidirectionnelle (SIMPLEX en point à point) du patient vers un récepteur situé à quelques mètres plus loin pour examen ou diagnostic par le spécialiste. Les informations à transmettre sont les signaux E.C.G. électrodes principales (E₁, E₂, E₃/Référence). Ces informations peuvent être transmises séquentiellement. Le récepteur est couplé à une table traçante (analogique ou numérique) et/ou à un enregistreur (analogique ou numérique). Chaque liaison patient/récepteur est sélective et correspond à une salle d'examen différente. Le nombre de liaisons simultanées nécessaires est au maximum de l'ordre de 10. La durée d'examen est en moyenne de 10 mn, l'autonomie souhaitée est de l'ordre de la journée (la recharge des accus est possible la nuit). Une utilisation comparable peut se trouver chez le vétérinaire pour les examens cardiologiques de chevaux de course notamment.

Pour une clinique cardiologique ou une maison de repos, les conditions sont un peu différentes : L'équipement permet une liaison unidirectionnelle (SIMPLEX en point à point) du patient surveillé vers un récepteur
- soit situé à quelques mètres plus loin dans la chambre du patient. Le récepteur est alors relié à un dispositif central d'enregistrement (muni éventuellement d'un système d'auto-diagonostic) par liaison bifilaire
- soit situé en un point NODAL et recevant l'ensemble des données transmises par les différentes liaisons : soit directement (la portée devant alors être importante) soit indirectement au moyen de balises relais.

Les informations à transmettre sont les signaux E.C.G. électrodes principales (E₁, E₂, E₃/référence). Ces informations peuvent être transmises séquentiellement. Le récepteur central est couplé à une table traçante (analogique ou numérique) et/ou à un enregistreur (analogique ou numérique). Le récepteur à courte portée correspond à une utilisation en chambre alors que le récepteur central (nodal) permet une surveillance du patient quelque soit sa position dans la clinique. Chaque liaison patient/récepteur est sélective. Le nombre de liaisons simultanées peut devenir supérieur à 10. Une liaison bidirectionnelle (DUPLEX) peut également être mise en oeuvre le retour permettant alors de transmettre une alarme vocale ou sonore (pour ordre de mise au repos, retour à la chambre pour examen ...). A défaut un mode alarme du type "pager" peut être très utile.

Les applications décrites ci-dessus ne sont pas limitatives. L'équipement selon l'invention peut être également utilisé dans les salles de sports.

## Revendications

1. Système de transmission de données médicales, sans fil pour la transmission entre émetteur/récepteur de signaux électriques caractéristiques résultant d'un champ électrique entre électrodes (E₁, E₂, E₃...) disposées sur le corps du patient, comprenant un équipement d'émission à maintenir sur le corps du patient par des moyens amovibles, relié à deux électrodes (E₁, E₂) et comportant un circuit (3) d'amplification des signaux électriques couplé à un modulateur (12) d'une porteuse à la fréquence d'émission (Fₚ), caractérisé en ce que, pour sécuriser le canal de transmission entre émetteur/récepteur et autoriser un fonctionnement multicanaux, l'équipement d'émission comporte un circuit codeur de canal (10) couplé d'une part à la sortie du circuit (3) d'amplification et d'autre part à un générateur de code (11) qui génère un code pseudo-aléatoire associé au canal de liaison affecté à l'équipement d'émission, pour coder le signal analogique utile avant transmission au modulateur (12).

2. Système selon la revendication 1, caractérisé en ce que le circuit codeur de canal (10) est couplé à la sortie du circuit (3) d'amplification par l'intermédiaire d'un convertisseur analogique numérique (6), d'un circuit de compression de débit (7), d'un circuit de codage correcteur d'erreurs (8) et d'un circuit d'empaquetage et d'entête (9) reliés dans cet ordre en série.

3. Système selon l'une quelconque des revendications 1 et 2, caractérisé en ce que le générateur de code (11) met en oeuvre des codes pseudo-aléatoires orthogonaux de type GOLD ou KASAMI, pour définir un nombre N de canaux séparables utilisant une même fréquence porteuse Fₚ.

4. Système selon l'une des revendications précédentes, caractérisé en ce qu'il comporte en outre une station de réception comportant un démodulateur (21) et une chaîne de traitement du signal démodulé pour la restitution du signal utile.

5. Système selon la revendication 4, caractérisé en ce que, l'équipement d'émission comportant un codeur de canal recevant directement le signal utile après le circuit (3) d'amplification avec commande automatique de gain, la chaîne de traitement du signal démodulé comporte un opérateur (22) relié d'une part à la sortie du démodulateur et d'autre part à la sortie d'un générateur de code canal (23), la sortie de cet opérateur étant reliée à l'entrée d'un sommateur (24) qui somme le signal reçu sur une séquence correspondant à la longueur de code et glissante à cadence du pseudo-aléatoire utilisé, un détecteur de maximum (25) et un sous-échantillonneur dont la sortie fournit le signal utile.

6. Système selon l'une quelconque des revendications 1 à 5, caractérisé en ce que l'équipement d'émission comporte un générateur de fréquence porteuse (Fₚ).

7. Système selon l'une quelconque des revendications 1 à 6, caractérisé en ce qu'il comprend interposé entre les électrodes (E₁, E₂) et le modulateur (12) un ensemble (1) d'amplification différentielle et de filtrage de bande également relié à une électrode de référence (Ref) pour filtrer la bande du signal utile tout en rejetant le mode commun, ainsi qu'un ensemble (2) de rejection sélective de la fréquence du secteur et de ses harmoniques dans la bande du signal utile.

## Claims

1. Wireless transmission system for medical data, for the transmission between sender/receiver of characteristic electric signals resulting from an electric field between electrodes (E₁, E₂, E₃...) placed on the body of a patient, comprising a piece of sending equipment to be held on the body of the patient by removable means, connected to two electrodes (E₁, E₂) and comprising a circuit (3) for amplifying the electric signals which is coupled to a modulator (12) of a carrier at the sending frequency (Fₚ), characterized in that, in order to render secure the transmission channel between sender/receiver and permit multichannel operation, the piece of sending equipment includes a channel coder circuit (10) coupled on the one hand to the output of the amplifying circuit (3) and on the other hand to a code generator (11) which generates a pseudo-random code associated with the linking channel assigned to the piece of sending equipment, in order to code the useful analogue signal before transmission to the modulator (12).

2. System according to Claim 1, characterized in that the channel coder circuit (10) is coupled to the output of the amplifying circuit (3) by way of an analogue/digital converter (6), a flow compression circuit (7), an error-correcting coding circuit (8) and a packeting and header circuit (9) which are connected in this order in series.

3. System according to either one of Claims 1 and 2, characterized in that the code generator (11) employs orthogonal pseudo-random codes of the GOLD or KASAMI type to define a number N of channels which can be separated using the same carrier frequency Fₚ.

4. System according to one of the preceding claims, characterized in that it furthermore comprises a receiving station comprising a demodulator (21) and a chain for processing the demodulated signal for the restoration of the useful signal.

5. System according to Claim 4, characterized in that, with the piece of sending equipment comprising a channel coder directly receiving the useful signal after the amplifying circuit (3) with automatic gain control, the chain for processing the demodulated signal comprises an operator (22) connected on the one hand to the output of the demodulator and on the other hand to the output of a channel code generator (23), the output of this operator being connected to the input of a summator (24) which sums the signal received on a sequence corresponding to the length of code and sliding at the rate of the pseudo-random used, a detector of maximum (25) and a subsampler whose output furnishes the useful signal.

6. System according to any one of Claims 1 to 5, characterized in that the piece of sending equipment comprises a carrier frequency (Fₚ) generator.

7. System according to any one of Claims 1 to 6, characterized in that it comprises interposed between the electrodes (E₁, E₂) and the modulator (12) a unit (1) for differential amplification and band filtering also connected to a reference electrode (Ref) to filter the band of the useful signal while rejecting the common mode, as well as a unit (2) for selective rejection of the frequency of the mains and of its harmonics in the band of the useful signal.

## Patentansprüche

1. System zur drahtlosen Übertragung von medizinischen Daten, das die Übertragung von charakteristischen elektrischen Signalen, die aus einem elektrischen Feld zwischen am Körper des Patienten befestigten Elektroden (E₁, E₂, E₃ ...) stammen, zwischen Sender und Empfänger bewirkt und das ein Sende-Gerät aufweist, das durch lösbare Mittel am Körper des Patienten gehalten wird, die mit zwei Elektroden (E₁, E₂) verbunden sind und eine verstärkungsschaltung (3) für die elektrischen Signale aufweisen, die mit einem Modulator (12) einer Trägerwelle auf Sendefrequenz (Fₚ) gekoppelt ist, dadurch gekennzeichnet, daß zur Sicherung des Übertragungskanals zwischen Sender und Empfänger und zur Ermöglichung eines Vielkanalbetriebs das Sende-Gerät eine Kanalkodierschaltung (10) aufweist, die einerseits an den Ausgang der Verstärkerschaltung (3) und andererseits an einen Kodegenerator (11) gekoppelt ist, der einen Pseudozufalls-Kode erzeugt, der dem dem Sende-Gerät zugehörigen Verbindungskanal zugeordnet ist, um das analoge Nutzsignal vor der Übertragung zum Modulator (12) zu kodieren.

2. System nach Anspruch 1, dadurch gekennzeichnet, daß die Kanalkodierschaltung (10) an den Ausgang der verstärkungsschaltung (3) über einen Analog-Digital-Wandler (6), eine Durchsatz-Kompressionsschaltung (7), eine Kodierschaltung (8) für einen Fehlerkorrekturkode und eine Schaltung (9) zur Paketbildung und zur Erzeugung eines Vorspanns gekoppelt ist, die in dieser Reihenfolge in Reihe geschaltet sind.

3. System nach einem beliebigen der Ansprüche 1 und 2, dadurch gekennzeichnet, daß der Kodegenerator (11) orthogonale pseudozufällige Kodes des Typs GOLD oder KASAMI verwendet, um eine Anzahl N von trennbaren Kanälen zu definieren, die die gleiche Trägerfrequenz Fₚ verwenden.

4. System nach einem der vorhergehenden Ansprüche 1 und 2, dadurch gekennzeichnet, daß es weiter eine Empfangsstation aufweist, die einen Demodulator (21) und eine Kette zur Verarbeitung des demodulierten Signals und zur Wiederherstellung des Nutzsignals enthält.

5. System nach Anspruch 4, dadurch gekennzeichnet, daß, wenn das Sendegerät einen Kanalkodierer aufweist, der direkt das Nutzsignal nach der Verstärkungsschaltung (3) mit automatischer Verstärkungssteuerung empfängt, die Verarbeitungskette für das demodulierte Signal einen Operator (22), der einerseits mit dem Ausgang des Demodulators und andererseits mit dem Ausgang eines Kanalkodegenerators (23) verbunden ist, wobei der Ausgang dieses Operators mit dem Eingang eines Summierglieds (24) verbunden ist, das das empfangene Signal über eine Sequenz summiert, die der Kodelänge entspricht und die mit dem Takt des verwendeten Pseudozufalls-Kodes gleitet, einen Maxima-Detektor (25) und ein Unter-Tastorgan aufweist, dessen Ausgang das Nutzsignal liefert.

6. System nach einem beliebigen der Ansprüche 1 bis 5, dadurch gekennzeichnet, daß das Sendegerät einen Generator für die Trägerfrequenz (Fₚ) aufweist.

7. System nach einem beliebigen der Ansprüche 1 bis 6, dadurch gekennzeichnet, daß es zwischen den Elektroden (E₁, E₂) und dem Modulator (12) eine Einheit (1) zur Differentialverstärkung und zur Bandfilterung aufweist, die auch mit einer Bezugselektrode (Ref) verbunden ist, um das Band des Nutzsignals zu filtern und zugleich den Gleichtakt-Modus zu unterdrücken, sowie eine Einheit (2) zur selektiven Unterdrückung des Netzfrequenz und ihrer Harmonischen im Frequenzband des Nutzsignals.
